# EUROPEAN PATENT SPECIFICATION

(11) **EP 4 129 304 B1**
(45) Date of publication and mention of the grant of the patent: **18.09.2024**
(21) Application number: 21781882.2
(22) Date of filing: 02.04.2021
(51) Int. Cl.: A61K 9/127, A61K 31/7068, A61P 35/00, A61K 9/10

(54) **ANTITUMOR AGENT**
ANTITUMORMITTEL
AGENT ANTITUMORAL

(30) Priority: 03.04.2020 US 202063004733 P; 18.05.2020 JP 2020086495
(43) Date of publication of application: 08.02.2023
(73) Proprietor: FUJIFILM Corporation, Tokyo 106-8620 (JP)
(72) Inventor: MATSUMOTO Takeshi, Ashigarakami-gun, Kanagawa 258-8577 (JP); KAKINUMA Chihaya, Ashigarakami-gun, Kanagawa 258-8577 (JP); MAKITA Keiko, Ashigarakami-gun, Kanagawa 258-8577 (JP)
(74) Representative: Hoffmann Eitle
(86) International application number: PCT/JP2021/014285
(87) International publication number: WO 2021/201267

(56) References cited:
- WO-A1-2015/166985
- WO-A1-2015/166985
- WO-A1-2017/078008
- WO-A1-2017/078008
- HIGUCHI T. ET AL.: "FF-10832 enables long survival via effective gemcitabine accumulation in a lethal murine peritoneal dissemination model", CANCER SCIENCE, vol. 110, no. 9, 1 September 2019 (2019-09-01), JP, pages 2933 - 2940, XP093040995, ISSN: 1347-9032, Retrieved from the Internet <URL:https://www.ncbi.nlm.nih.gov/pmc/articles/PMC6726679/pdf/CAS-110-2933.pdf> DOI: 10.1111/cas.14123
- CLINICAL TRIAL NCT03440450: "A Phase 1 Dose-escalation Study of FF-10832 for Treatment of Solid Tumors - Full Text View - ClinicalTrials.gov", 1 January 2018 (2018-01-01), XP093040988, Retrieved from the Internet <URL:https://clinicaltrials.gov/ct2/show/study/NCT03440450> [retrieved on 20230421]
- FDA (US), CDER: "Estimating the Maximum Safe Starting Dose in Initial Clinical Trials for Therapeutics in Adult Healthy Volunteers", GUIDANCE FOR INDUSTRY, 1 July 2005 (2005-07-01), XP055152598
- ANONYMOUS: "News Release", FUJIFILM - NEWS RELEASE, 12 November 2018 (2018-11-12), XP009540558, Retrieved from the Internet <URL:https://www.fujifilm.co.jp/corporate/news/articleffnr_1366.html>
- ELI LILLY JAPAN K.K.: "Clinical practice guidelines for pancreatic cancer 2019", JAPANESE PANCREAS SOCIETY, pages 1 - 2, XP055966720, Retrieved from the Internet <URL:https://minds.jcqhc.or.jp/n/med/4/med0037/G0001105> [retrieved on 20220930]
- BORAZANCI E. H., GERALD STEVEN FALCHOOK, ATIF ABBAS, CATHERINE A. WHEELER, GARY MAIER, MARY JOHANSEN: "A phase I dose-escalation and immune biomarker study of intravenous FF-10832, liposomal gemcitabine, in patients with advanced solid tumors", JOURNAL OF CLINICAL ONCOLOGY. 2019 ASCO ANNUAL MEETING I, vol. 37, no. 15 suppl., 1 January 2019 (2019-01-01), pages TPS3163, XP055924062, DOI: 10.1200/JC0.2019.37.15_suppl

## Description

### Technical Field

The present invention relates to an anti-tumor agent in which a liposome encompasses gemcitabine or a salt thereof, where the anti-tumor agent is administered according to a specific amount and a specific schedule.

### Background Art

Gemcitabine has been used as a useful drug in chemotherapy for malignant tumors. Gemcitabine has a metabolic antagonism that inhibits DNA synthesis. Since a drug having a metabolic antagonism attacks only a part of cells in the DNA synthesis phase, the effective cell-killing property cannot be obtained in a case where the exposure time is short. In a case where the metabolism of such a drug in the body after being administered is rapid, sufficient exposure time to the tumor cannot be obtained, and the expected drug efficacy is not obtained in a large number of cases.

It is known that by encompassing a drug in an inner water phase of a liposome in a state of being dissolved therein and setting a liposome composition under hypertonic conditions, the release of the drug from the liposome composition can be set at a proper rate, and more suitable drug delivery can be realized (Patent Documents 1 and 2).

Patent Documents 1, 2 and 3 have reported liposome compositions with which gemcitabine is encompassed in liposomes for a time sufficient for the exposure of gemcitabine in the tumor. Further, Patent Documents 4 and 5 have been reported production methods for a liposome composition with which gemcitabine is encompassed in liposomes.

### Prior Art Documents

### Patent Documents

Patent Document 1: WO2015/166985A
Patent Document 2: WO2015/166986A
Patent Document 3: WO2015/166987A
Patent Document 4: WO2015/166988A
Patent Document 5: WO2017/078009A

### SUMMARY OF THE INVENTION

So far, there have been no reports on the results of the administration of liposomes encompassing gemcitabine or a salt thereof to cancer patients. As a result, it is not known what kind of blood kinetics the liposomes encompassing gemcitabine or a salt thereof exhibit. Even a person skilled in the art cannot estimate what dose rate makes it possible to achieve that liposomes encompassing gemcitabine or a salt thereof exhibit the drug efficacy of the gemcitabine or salt thereof to cancer patients unless actually administering them to cancer patients. In addition, the value of blood concentration of the liposomes encompassing gemcitabine or a salt thereof, required to exhibit an anti-tumor effect, has not been studied so far.

An object of an aspect of the present invention is to provide an anti-tumor agent that exhibits a remarkably excellent anti-tumor effect.

As a result of diligent studies to solve the above problems, the inventors of the present invention found that a particularly excellent anti-tumor effect can be obtained in a case of being administered at a specific dose rate and schedule, thereby completing the present invention.

That is, the present invention provides the following aspects.
<1> An anti-tumor agent for use in curing cancer in a human, the anti-tumor agent comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol,
   the gemcitabine or the salt thereof encompassed in the liposome is administered at a dose rate of 40 mg/m² body surface area to 60 mg/m² body surface area in terms of gemcitabine per administration, and
   the anti-tumor agent is repeatedly administered a plurality of times of single administration at every three weeks to four weeks.
<2> The anti-tumor agent for use according to <1>, in which a formulation form is a liquid medicinal preparation, and a concentration of the gemcitabine or the salt thereof is 0.01 mg/mL to 10 mg/mL in terms of gemcitabine.
<3> The anti-tumor agent for use according to <1> or <2>, in which the cancer is an advanced solid cancer.
<4> The anti-tumor agent for use according to <3>, in which the solid cancer is at least one selected from pancreatic cancer, uterine cancer, appendix cancer, ovarian cancer, lung cancer, breast cancer, biliary tract cancer, bladder cancer, colon cancer, gastric cancer, or non-Hodgkin lymphoma.
<5> The anti-tumor agent for use according to any one of <1> to <4>, in which an average particle diameter of the liposomes is 5 nm to 100 nm.
<6> The anti-tumor agent for use according to any one of <1> to <5>, in which in terms of a blending rate to a whole of the lipid constituting the liposome, the hydrogenated soybean phosphatidylcholine has a blending rate of 50% by mass to 90% by mass, the 1,2-distearoyl-3-phosphatidylethanolamine-polyethylene glycol has a blending rate of 1% by mass to 50% by mass, and the cholesterol has a blending rate of 1% by mass to 20% by mass.
<7> The anti-tumor agent for use according to any one of <1> to <6>, in which an administration route is an intravenous administration.
<8> The anti-tumor agent for use according to any one of <1> to <7>, in which the anti-tumor agent is administered by infusion for 15 minutes to 240 minutes in a single administration.
<9> An anti-tumor agent for use in curing cancer in a human, the anti-tumor agent comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 28 days, in which on 1st day, an amount of the gemcitabine or the salt thereof encompassed in the liposome is administered at a dose rate of 40 mg/m² body surface area to 60 mg/m² body surface area in terms of gemcitabine per administration and the administration is discontinued from 2nd day to 27th day, is repeated.
<10> An anti-tumor agent for use in curing cancer in a human, the anti-tumor agent comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 21 days, in which on 1st day, an amount of the gemcitabine or the salt thereof encompassed in the liposome is administered at a dose rate of 40 mg/m² body surface area to 60 mg/m² body surface area in terms of gemcitabine per administration and the administration is discontinued from 2nd day to 20th day, is repeated.

In addition, the following items <A> to <O> are disclosed herein.
<A> A treatment method for curing cancer, comprising:
   administering, to a subject, an anti-tumor agent that contains a liposome containing an inner water phase, and an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   the gemcitabine or the salt thereof encompassed in the liposome is administered to the subject at a dose rate of 1.0 mg/m² body surface area to 240 mg/m² body surface area, in terms of gemcitabine per administration.
<B> An anti-tumor agent for using in curing of cancer, comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   the gemcitabine or the salt thereof encompassed in the liposome is administered at a dose rate of 1.0 mg/m² body surface area to 240 mg/m² body surface area, in terms of gemcitabine per administration.
<C> Use of a composition, which is the use of a composition for producing an anti-tumor agent for curing cancer, the use comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   the gemcitabine or the salt thereof encompassed in the liposome is administered at a dose rate of 1.0 mg/m² body surface area to 240 mg/m² body surface area, in terms of gemcitabine per administration.
<D> A treatment method for curing cancer, comprising:
   administering, to a subject, an anti-tumor agent that contains a liposome containing an inner water phase, and an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 28 days, in which on 1st day and 15th day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 14th day and from 16th day to 27th day, is repeated.
<E> An anti-tumor agent for using in curing of cancer, the anti-tumor agent comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 28 days, in which on 1st day and 15th day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 14th day and from 16th day to 27th day, is repeated.
<F> Use of a composition, which is the use of a composition for producing an anti-tumor agent for curing cancer, the use comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 28 days, in which on 1st day and 15th day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 14th day and from 16th day to 27th day, is repeated.
<G> A treatment method for curing cancer, comprising:
   administering, to a subject, an anti-tumor agent that contains a liposome containing an inner water phase, and an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 21 days, in which on 1st day and 8th day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 7th day and from 9th day to 20th day, is repeated.
<H> An anti-tumor agent for using in curing of cancer, the anti-tumor agent comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 21 days, in which on 1st day and 8th day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 7th day and from 9th day to 20th day, is repeated.
<I> Use of a composition, which is the use of a composition for producing an anti-tumor agent for curing cancer, the use comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 21 days, in which on 1st day and 8th day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 7th day and from 9th day to 20th day, is repeated.
<J> A treatment method for curing cancer, comprising:
   administering, to a subject, an anti-tumor agent that contains a liposome containing an inner water phase, and an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 28 days, in which on 1st day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 27th day, is repeated.
<K> An anti-tumor agent for using in curing of cancer, the anti-tumor agent comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 28 days, in which on 1st day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 27th day, is repeated.
<L> Use of a composition, which is the use of a composition for producing an anti-tumor agent for curing cancer, the use comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 28 days, in which on 1st day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 27th day, is repeated.
<M> A treatment method for curing cancer, comprising:
   administering, to a subject, an anti-tumor agent that contains a liposome containing an inner water phase, and an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 21 days, in which on 1st day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 20th day, is repeated.
<N> An anti-tumor agent for using in curing of cancer, the anti-tumor agent comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 21 days, in which on 1st day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 20th day, is repeated.
<O> Use of a composition, which is the use of a composition for producing an anti-tumor agent for curing cancer, the use comprising:
   a liposome containing an inner water phase; and
   an aqueous solution dispersing the liposome, which constitutes an outer water phase,
   in which the liposome encompasses gemcitabine or a salt thereof,
   a lipid constituting the liposome contains at least hydrogenated soybean phosphatidylcholine, 1,2-distearoyl-3-phosphatidylethanolamine-polyethylene glycol, and cholesterol, and
   a cycle as one cycle of 21 days, in which on 1st day, an amount of the gemcitabine or the salt thereof encompassed in the liposome is administered at a dose rate of 1.0 mg/m² body surface area to 240 mg/m² body surface area in terms of gemcitabine per administration and the administration is discontinued from 2nd day to 20th day, is repeated.

According to the anti-tumor agent of the aspect of the present invention, an excellent anti-tumor effect is obtained, and the effect of reducing the volume of cancer is remarkable. In addition, as a result of the anti-tumor effect obtained from the anti-tumor agent according to the aspect of the present invention, it is expected that the progression-free survival time and the overall survival time are extended.

### DESCRIPTION OF THE PREFERRED EMBODIMENTS

In the present invention, the range represented by "to" includes the values at both ends thereof unless otherwise specified. In the present invention, the "tumor" is used synonymously with "malignant tumor" and "cancer". The "malignant tumor" means a tumor in which the morphology of tumor cells and the arrangement thereof are different from those of the normal cells from which the tumor cells are derived and which is invasive or metastatic. The "treatment" means curing each disease. The "subject" is a mammal such as a human, a mouse, a monkey, or a domestic animal requiring prevention or curing therefor, and preferably a human requiring prevention or curing therefor. The "prevention" means the inhibition of the onset of a disease, the reduction of the risk of the onset of a disease, or the delay of the onset of a disease. The "curing" means the amelioration or the suppression (the maintenance or delay) of the progression of a disease or state. The "progression-free survival time" means the time during which cancer has not progressed and remained in a stable state during curing (after curing). The "overall survival time" means the time during which a subject has survived from the allocation start date of therapy or curing start date in a clinical test.

Hereinafter, the present invention will be described in detail. The present invention is an anti-tumor agent for curing cancer, the anti-tumor agent containing a liposome containing an inner water phase and an aqueous solution dispersing the liposome, which constitutes an outer water phase, in which the liposome encompasses gemcitabine or a salt thereof, a lipid constituting the liposome contains at least hydrogenated soybean phosphatidylcholine, 1,2-distearoyl-3-phosphatidylethanolamine-polyethylene glycol, and cholesterol, and the gemcitabine or the salt thereof encompassed in the liposome is administered at a dose rate of 40 mg/m² body surface area to 60 mg/m² body surface area in terms of gemcitabine per administration.

### (Liposome)

A liposome is a closed vesicle formed of a lipid bilayer membrane using a lipid, and an aqueous phase (an inner water phase) is included in the space of the closed vesicle. The inner water phase contains water. The liposome is generally present in a state of being dispersed in an aqueous solution (an outer water phase) outside the closed vesicle. The liposome may be a single lamella (which is also called a single-layer lamella or uni-lamella, where the bilayer membrane has a single layered structure) or may be a multi-layer lamella (which is also called a multi-lamella and has a structure of a large number of bilayer membranes, having an onion-like shape, where the individual layers are separated by an aqueous layer). However, in the present invention, a single lamellar liposome is preferable from the viewpoint of safety and stability in pharmaceutical use applications.

The form of the liposome is not particularly limited as long as the liposome is a liposome capable of encompassing a drug. The "encompassing" means taking a form in which a drug is contained in the inner water phase of the liposome. Examples thereof include a form in which a drug is enclosed in a closed space formed of a membrane and a form in which a drug is encompassed in the membrane itself, where a combination of these may be good.

The size (average particle diameter) of the liposomes is not particularly limited; however, it is 2 to 200 nm, preferably 5 to 150 nm, more preferably 5 to 120 nm, and still more preferably 5 to 100 nm. In a case of expecting the enhanced permeation and retention effect (the EPR effect) described below, regarding the size (average particle diameter) of the liposomes, the diameter is preferably substantially 50 to 200 nm, the diameter is more preferably substantially 50 to 150 nm, and the diameter is still more preferably substantially 50 to 100 nm. The term "substantially" means that at least 75% of the number of liposomes is within the specified diameter range. The above-described "at least 75%" is more preferably at least 80% and still more preferably at least 90%.

In the present invention, the "average particle diameter" means an average particle diameter (preferably, a cumulant average particle diameter) measured by using a dynamic light scattering method unless otherwise specified. The "average particle diameter" can be measured by using a device that can measure the average particle diameter according to a light scattering method.

The component constituting the lipid bilayer membrane of the liposome is selected from lipids. A lipid constituting the liposome according to the embodiment of the present invention is hydrogenated soybean phosphatidylcholine, 1,2-distearoyl-3-phosphatidylethanolamine-polyethylene glycol, and cholesterol.

In terms of the blending rate to the whole of the lipid constituting the liposome according to the embodiment of the present invention, it is preferable that the hydrogenated soybean phosphatidylcholine has a blending rate of 50% by mass to 90% by mass, the 1,2-distearoyl-3-phosphatidylethanolamine-polyethylene glycol has a blending rate of 1% by mass to 50% by mass, and the cholesterol has a blending rate of 1% by mass to 20% by mass, and it is more preferable that the hydrogenated soybean phosphatidylcholine has a blending rate of 55% by mass to 80% by mass, the 1,2-distearoyl-3-phosphatidylethanolamine-polyethylene glycol has a blending rate of 10% by mass to 30% by mass, and the cholesterol has a blending rate of 1% by mass to 10% by mass.

### (Gemcitabine or salt thereof)

The liposome according to the embodiment of the present invention encompasses gemcitabine or a salt thereof. Gemcitabine has a chemical name of (+)-2'-deoxy-2',2'-difluorocystidine, and it is an anti-cancer agent having a metabolic antagonism. In the present invention, the gemcitabine may be gemcitabine itself or may be a salt thereof, which is acceptable as a pharmaceutical product, or it may be a prodrug that liberates gemcitabine in vivo. In the present invention, it is preferable to use gemcitabine hydrochloride.

The gemcitabine encompassed in the liposome according to the embodiment of the present invention is present in a state of being dissolved in the inner water phase of the liposome. Here, the state of being dissolved is regarded as a state of being encompassed in the state of being dissolved, in a case where the amount of the drug filled with respect to the volume of the liposome is equal to or smaller than the saturated solubility of the drug in the composition solution of the inner water phase.

Further, in a case where no drug crystal is observed in Cryo-TEM or no diffraction pattern due to the crystal lattice is observed in the XRD measurement even at the saturated solubility or higher solubility, it is regarded that gemcitabine is encompassed in the state of being dissolved.

### (Production method for liposome)

The liposome according to the embodiment of the present invention is not particularly limited as long as it can be produced by a method that enables the production of a liposome composition with which gemcitabine is encompassed in the liposome, in a state of being dissolved. However, the production thereof can be realized with referenced to, for example, WO2015/166985A and WO2015/166986A.

In the liposome according to the embodiment of the present invention, the osmotic pressure of the inner water phase is 2 to 8 times, preferably 2.5 to 6 times, and more preferably 3 to 5 times the osmotic pressure of the outer water phase. In a case of setting the osmotic pressure of the inner water phase of the liposome to 2 to 8 times the osmotic pressure of the outer water phase, it is possible to obtain a liposome that can achieve both ease of drug release and drug storage stability.

The osmotic pressures of the inner water phase of the liposome and the outer water phase can be measured, for example, as follows, which are not limited thereto. In the liposome manufacturing steps described in WO2015/166985A and WO2015/166986A, the solutes of the outer water phase and the inner water phase in the liquid obtained after the final drug loading step are homogenized, and thus the osmotic pressure at that time can be measured, thereby defining it as the osmotic pressure of the inner water phase of the liposome. However, it is limited to a case where the solute of the inner water phase is sufficiently retained, for example, a case where the heating operation is suppressed so that the phase transition of the lipid does not occur in the subsequent replacement and osmotic pressure adjustment step by dialysis of the outer water phase. In addition, the osmotic pressure of the outer water phase can be defined by measuring the osmotic pressure of the dialysate that is used in the final dialysis step. However, it is limited to a case where the replacement with the dialysate can be sufficiently carried out. Further, the osmotic pressures of the inner water phase and the outer water phase can be also defined by quantifying the composition concentration of the outer water phase and the solute composition concentration of the inner water phase in the finished solution of the liposome by using centrifugation or ultrafiltration and measuring the osmotic pressure of the composition solution.

It suffices that the osmotic pressure is measured according to the osmotic pressure measuring method described in the Japanese Pharmacopoeia, Sixteenth Edition. Specifically, the osmolar concentration can be determined by measuring the degree of the drop of the solidifying point (the freezing point) of water. Further, the degree of the drop of the solidifying point of water is defined in terms of a molar concentration of a solute, and thus the osmolar concentration can be also determined from the molar concentration of the solute.

The osmotic pressure of the outer water phase of the liposome according to the embodiment of the present invention has an important effect on the living body during administration. In a case where it is far from the osmotic pressure of the body fluid, hemolysis and pain due to the movement of water in each tissue occur. For this reason, the osmotic pressure of the outer water phase in the present invention is preferably 200 to 400 mOsmol/L, more preferably 250 to 350 mOsmol/L, and most preferably isotonic to the body fluid.

### (Release rate)

The release rate means the amount of drug (here, gemcitabine) per unit time, which goes out of the liposome. In the liposome according to the embodiment of the present invention, the release rate at 37°C in plasma is preferably 10% by mass/24hr or more and 70% by mass/24hr or less, more preferably 20% by mass/24hr or more and 60% by mass/24hr or less, and still more preferably 20% by mass/24hr or more and 50% by mass/24hr or less.

Since the release rate depends on the temperature, it is preferable to measure it under the constant temperature conditions. For example, although the temperature is not particularly limited in a case of a human, it is preferable to measure it within the range of body temperature (35°C or higher and 38°C or lower).

In a case where the release rate is less than 10% by mass/24 hr, it is not possible to obtain a sufficient exposure time in the body as an anti-tumor agent, and the expected drug efficacy cannot be obtained in a large number of cases. In addition, in some cases, liposomes may remain in the body for an unnecessarily long period of time and accumulate in tissues such as skin which the liposomes are difficult to distribute, which results in unexpected toxicity. In a case where it is more than 70% by mass/24 hr, the amount of the drug exposed per unit time increases, and thus the maximum blood concentration of the drug increases, thereby increasing the toxicity, and the leaked drug distributes to tissues other than the tumor site or is rapidly metabolized, which is not preferable since the retention in the blood decreases.

### (Anti-tumor agent)

According to the present invention, an anti-tumor agent for curing cancer is provided.

The cancer in the present invention is preferably an advanced solid cancer. Cancer is basically classified into 5 stages from a stage 0 to a stage IV according to the degree of progression. In a case of being referred to as advanced, it is generally, but not limited to, a stage III or higher. In addition, the solid cancer is more preferably at least one selected from pancreatic cancer, uterine cancer, appendix cancer, ovarian cancer, lung cancer, biliary tract cancer, bladder cancer, colon cancer, gastric cancer, non-Hodgkin lymphoma, breast cancer, or a sarcoma, still more preferably pancreatic cancer, uterine cancer, appendix cancer, lung cancer, bladder cancer, liposarcoma, malignant melanoma, Hodgkin's lymphoma, renal cell cancer, or esophageal cancer, and most preferably, pancreatic cancer, lung cancer, bladder cancer, or biliary tract cancer.

Examples of the lung cancer include non-small cell lung cancer (adenocarcinoma, squamous cell carcinoma, and large cell carcinoma) and small cell lung cancer.

Examples of the bladder cancer include urothelial carcinoma, squamous cell carcinoma, and adenocarcinoma.

The anti-tumor agent according to the embodiment of the present invention is preferably used in the curing of cancer, for which therapy using gemcitabine is effective, and it is more preferably used in the curing of cancer resistant to gemcitabine.

The resistance means that cancer cells exhibit tolerance (resistance) to anti-cancer agents, which refers to the natural resistance that anti-cancer agents do not work from the beginning of curing and a state where the effect of anti-cancer agents effective in the beginning is not exhibited as the curing is continued or the effect thereof is attenuated. Specifically, it means properties of showing no proper response to an anti-cancer agent from the viewpoint that a response to an anti-cancer agent is shown in the initial stage but responsiveness is reduced during the subsequent curing or cells continue to proliferate in the process of the curing using the anti-cancer agent.

The formulation form of the anti-tumor agent according to the embodiment of the present invention is preferably a liquid medicinal preparation, and examples thereof include an injection agent.

The concentration of gemcitabine or a salt thereof contained in the liquid medicinal preparation according to the embodiment of the present invention is preferably 0.01 mg/mL to 10 mg/mL in terms of gemcitabine. It is more preferably 0.1 mg/mL to 5 mg/mL and still more preferably 0.1 mg/mL to 1 mg/mL.

The liquid medicinal preparation according to the embodiment of the present invention generally may contain additives such as an emulsifying agent, a surfactant, a dissolution assisting agent, a suspending agent, an isotonizing agent, a buffering agent, a preservative, an antioxidant, a stabilizer, and an absorption promoting agent.

The isotonizing agent is not particularly limited. However, examples thereof include inorganic salts such as sodium chloride, potassium chloride, sodium hydrogen phosphate, sodium dihydrogen phosphate, and potassium dihydrogen phosphate; polyols such as glycerol, mannitol, and sorbitol; and sugars such as glucose, fructose, lactose, and sucrose.

The stabilizer is not limited to; however, examples thereof include sugars such as glycerol, mannitol, sorbitol, lactose, and sucrose.

The antioxidant is not particularly limited; however, examples thereof include ascorbic acid, uric acid, tocopherol homologues (for example, vitamin E and four isomers of tocopherol α, β, γ, and δ), cysteine, and EDTA. The stabilizer and the antioxidant can be each used alone or in a combination of two or more.

Examples of the pH adjusting agent include sodium hydroxide, citric acid, acetic acid, triethanolamine, sodium hydrogen phosphate, sodium dihydrogen phosphate, and potassium dihydrogen phosphate.

The liquid medicinal preparation according to the embodiment of the present invention may contain a pharmaceutically acceptable organic solvent, collagen, polyvinyl alcohol, polyvinylpyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, casein, gelatin, agar, diglycerin, propylene glycol, polyethylene glycol, vaseline, paraffin, stearyl alcohol, stearic acid, human serum albumin (HSA), mannitol, sorbitol, lactose, PBS, sodium chloride, sugars, an in vivo degradable polymer, a serum-free medium, and an additive acceptable as a pharmaceutical additive.

In particular, the liquid medicinal preparation according to the embodiment of the present invention preferably contains sucrose, L-histidine, sodium chloride, and sodium hydroxide in the outer water phase.

The pH of the outer water phase of the liquid medicinal preparation according to the embodiment of the present invention is preferably neutral, and specifically, it is preferably a pH of about 5.5 to 8.5.

The administration method for the anti-tumor agent according to the embodiment of the present invention is preferably parenteral administration. The administration route includes routes of intravenous, intraarterial, intramuscular, intraperitoneal, subcutaneous, intraocular, and intraspinal, where a route of intravenous is preferable. Examples of the administration method include administration with a syringe or drip infusion.

The container to be filled with the liquid medicinal preparation is not particularly limited; however, it is preferably made of a material having low oxygen permeability. Examples thereof include a plastic container, a glass container, and a bag made of a laminated film, which has, as a gas barrier layer, an aluminum foil, an aluminum vapor-deposited film, an aluminum oxide vapor-deposited film, a silicon oxide vapor-deposited film, polyvinyl alcohol, an ethylene vinyl alcohol copolymer, polyethylene terephthalate, polyethylene naphthalate, or polyvinylidene chloride. As necessary, a bag using colored glass, an aluminum foil, or an aluminum vapor-deposited film can be adopted to shield light.

In the container filled with the liquid medicinal preparation, it is preferable to replace the gas in the container space part and the chemical liquid with an inert gas such as nitrogen in order to prevent the oxidation due to oxygen present in the space part inside the container. For example, an injection solution is subjected to nitrogen bubbling and a container is filled with the injection solution in a nitrogen atmosphere.

The anti-tumor agent according to the embodiment of the present invention can also be used in combination with another active substance or a treatment method, which is useful in curing the cancer of interest. It can be used in combination with, as the treatment method, physical curing such as radiotherapy or particle beam therapy, surgical curing such as surgery, chemotherapy, molecule targeted therapy, and cancer immunotherapy. It can be used in combination with, as the other active substance, a chemotherapeutic agent that is used in chemotherapy, a molecule targeted therapeutic drug that is used in molecule targeted curative medicine, a cell preparation or antibody preparation which is used in cancer immunotherapy, and an immune checkpoint inhibitor. Examples of the chemotherapeutic agent include an alkylating agent, an antimetabolite, an anti-tumor antibiotic, an alkaloid, a hormone therapeutic agent, a platinum complex, an angiogenesis inhibitor, a topoisomerase inhibitor, and a microtubule targeted agent.

### (Dosage form and dose rate)

In the anti-tumor agent according to the embodiment of the present invention, the dose per administration of the gemcitabine or the salt thereof encompassed in the liposome is such that the dose rate is 40 mg/m² body surface area to 60 mg/m² body surface area in terms of gemcitabine.

The anti-tumor agent according to the embodiment of the present invention is repeatedly administered a plurality of times of single administration at every three weeks to four weeks.

The anti-tumor agent according to the embodiment of the present invention is preferably administered by infusion over 15 to 240 minutes in a single administration. 15 minutes to 180 minutes are more preferable, 30 minutes to 150 minutes are still more preferable, and 30 minutes to 120 minutes are most preferable.

Another aspect of the present invention is an anti-tumor agent containing a liposome containing an inner water phase and an aqueous solution dispersing the liposome, which constitutes an outer water phase, in which the liposome encompasses gemcitabine or a salt thereof, a lipid constituting the liposome contains at least hydrogenated soybean phosphatidylcholine, 1,2-distearoyl-3-phosphatidylethanolamine-polyethylene glycol, and cholesterol, and a cycle as one cycle of 28 days, in which on 1st day and 15th day, an amount of the gemcitabine or the salt thereof encompassed in the liposome is administered at a dose rate of 40 mg/m² body surface area to 60 mg/m² body surface area in terms of gemcitabine per administration and the administration is discontinued from 2nd day to 14th day and from 16th day to 27th day, is repeated.

The preferred embodiment thereof is the same as the above-described contents.

The dose rate per administration of gemcitabine or a salt thereof encompassed in the liposome is, for example, about 40 mg/m² body surface area, about 55 mg/m² body surface area, or about 60 mg/m² body surface area in terms of gemcitabine.

Still another aspect of the present invention is an anti-tumor agent containing a liposome containing an inner water phase and an aqueous solution dispersing the liposome, which constitutes an outer water phase, in which the liposome encompasses gemcitabine or a salt thereof, a lipid constituting the liposome contains at least hydrogenated soybean phosphatidylcholine, 1,2-distearoyl-3-phosphatidylethanolamine-polyethylene glycol, and cholesterol, and a cycle as one cycle of 21 days, in which on the 1st day and the 8th day, an amount of the gemcitabine or the salt thereof encompassed in the liposome is administered at a dose rate of 40 mg/m² body surface area to 60 mg/m² body surface area in terms of gemcitabine per administration and the administration is discontinued from the 2nd day to the 7th day and from the 9th day to the 20th day, is repeated.

The dose rate per administration of gemcitabine or a salt thereof encompassed in the liposome is, for example, about 40 mg/m² body surface area, about 55 mg/m² body surface area, or about 60 mg/m² body surface area in terms of gemcitabine.

The preferred embodiment thereof is the same as the above-described contents.

Another aspect of the present invention is an anti-tumor agent containing a liposome containing an inner water phase and an aqueous solution dispersing the liposome, which constitutes an outer water phase, in which the liposome encompasses gemcitabine or a salt thereof, a lipid constituting the liposome contains at least hydrogenated soybean phosphatidylcholine, 1,2-distearoyl-3-phosphatidylethanolamine-polyethylene glycol, and cholesterol, and a cycle as one cycle of 28 days, in which on the 1st day, an amount of the gemcitabine or the salt thereof encompassed in the liposome is administered at a dose rate of 40 mg/m² body surface area to 60 mg/m² body surface area in terms of gemcitabine per administration and the administration is discontinued from the 2nd day to the 27th day, is repeated.

The preferred embodiment thereof is the same as the above-described contents.

The dose rate per administration of gemcitabine or a salt thereof encompassed in the liposome is in terms of gemcitabine, for example, about 40 mg/m² body surface area, about 55 mg/m² body surface area, or about 60 mg/m² body surface area.

The dose rate per administration of gemcitabine or a salt thereof encompassed in the liposome is preferably about 40 mg/m² body surface area to about 60 mg/m² body surface area and more preferably about 55 mg/m² body surface area in terms of gemcitabine.

Another aspect of the present invention is an anti-tumor agent containing a liposome containing an inner water phase and an aqueous solution dispersing the liposome, which constitutes an outer water phase, in which the liposome encompasses gemcitabine or a salt thereof, a lipid constituting the liposome contains at least hydrogenated soybean phosphatidylcholine, 1,2-distearoyl-3-phosphatidylethanolamine-polyethylene glycol, and cholesterol, and a cycle as one cycle of 21 days, in which on the 1st day, an amount of the gemcitabine or the salt thereof encompassed in the liposome is administered at a dose rate of 40 mg/m² body surface area to 60 mg/m² body surface area in terms of gemcitabine per administration and the administration is discontinued from the 2nd day to the 20th day, is repeated.

The preferred embodiment thereof is the same as the above-described contents.

The dose rate per administration of gemcitabine or a salt thereof encompassed in the liposome is in terms of gemcitabine, for example, about 40 mg/m² body surface area, about 55 mg/m² body surface area, or about 60 mg/m² body surface area.

The dose rate per administration of gemcitabine or a salt thereof encompassed in the liposome is preferably about 40 mg/m² body surface area to about 60 mg/m² body surface area and more preferably about 55 mg/m² body surface area in terms of gemcitabine.

### Examples

The present invention will be described in more detail below according to Examples; however, the present invention is not limited to these Examples.

### <Preparation of liquid medicinal preparation containing gemcitabine-encompassing liposome>

With reference to WO2015/166985A, a liquid medicinal preparation (hereinafter, referred to as A preparation) containing gemcitabine-encompassing liposomes having the following composition was prepared.
Gemcitabine hydrochloride: 0.57 mg/mL
Hydrogenated soybean phosphatidylcholine: 11.3 mg/mL
MPEG-DSPE (Note 1): 2.91 mg/mL
Cholesterol: 1.39 mg/mL
Sucrose: 94 mg/mL
L-histidine: 1.55 mg/mL
Sodium chloride: 0.188 mg/mL
pH adjusting agent: appropriate amount
(Note 1) N-(carbonyl-methoxypolyethylene glycol 2000)-1,2-distearoyl-sn-glycerol-3-phosphoethanolamine sodium salt

Gemcitabine hydrochloride was obtained from Teva Pharmaceutical Industries Ltd. API Division, and hydrogenated soybean phosphatidylcholine and MPEG-DSPE were obtained from NOF Corporation. Regarding other reagents, commercially available products in accordance with the United States Pharmacopeia were used.

### <Physical property value of A preparation>

The osmotic pressure of the inner water phase of the liposome was 3.8 times the osmotic pressure of the outer water phase.

The release rate of gemcitabine from the liposome was 25% by mass/24 hr in human plasma at 37°C, and the average particle diameter thereof was 77 nm.

The A preparation was infused into a 10 mL glass vial and used in the curing. It is noted that the concentration of gemcitabine hydrochloride in the preparation infused into the vial was 0.57 mg/mL, and the pH thereof was 6.5 to 8.0.

### <Reference Example 1: Prediction of dose>

The A preparation was diluted with a 5% glucose solution to 0.1, 0.2, and 0.3 mg/mL, and intravenous administration at 1, 2, and 3 mg/kg (respectively, 6, 12, and 18 mg/m²) was carried out once a week for four weeks to 15 male and 15 female SD rats (Charles River Laboratories Japan, Inc.) (30 rats in total). At 2 mg/kg or less, there was no death due to the A preparation during the test period. At 3 mg/kg, a total of 6 males and females (20%) were found dead or sacrificed in extremis during the administration period. The cause of death or sacrifice in extremis was degenerative necrosis or atrophy of the gastrointestinal epithelium, myelosuppression, and sepsis due to myelosuppression. As changes associated with the preparation A in planned autopsy individuals, dose rate-responsive myelosuppression and reduced feeding amount at 1 mg/kg or more, thymic atrophy, degenerative necrosis or atrophy of the gastrointestinal epithelium, and degenerative necrosis or atrophy of the spermatogenic epithelium were observed, which were conceived to change in response to the cell growth inhibitory effect of the A preparation. Based on the above results, the maximum tolerated dose rate was set to 2 mg/kg (12 mg/m²) at which no death is observed, and the initial human dose rate was set to 1.2 mg/m².

Based on the dose predicted from Reference Example 1, the A preparation was used in the curing shown in Examples 1 and 2 below. It is noted that the curing was carried out at Honor Health Research Institute located in Scottsdale, Arizona, USA, Sarah Cannon Research Institute located in Denver, Colorado, USA, University of Texas, M.D. Anderson Cancer Center, located in Houston, Texas, USA, and Sarah Cannon Research Institute located in Nashville, Texas, USA.

### <Example 1 (Reference): Administration test 1>

The administration cycle of administering the A preparation to a cancer patient once every two weeks was repeated. Specifically, 28 days were set as one cycle, the A preparation was administered on the 1st day and the 15th day, and the cycle consisting of these 28 days was repeated.

The curing effect was determined according to the following criteria.

The subject to be evaluated was checked by image diagnosis by computed tomography (CT) or magnetic resonance imaging (MRI), and the evaluation was made according to the following criteria.
Complete response (CR): A state in which the tumor is completely disappeared.
Partial response (PR): A state in which the sum of the sizes of the tumor is reduced by 30% or more.
Stable disease (SD): A state in which the size of the tumor does not change.
Progressive disease (PD): A state in which the sum of the sizes of the tumor size is increased by 20% or more and the absolute value thereof is increased by 5 mm or more or a state in which a new lesion has appeared.

It is noted that since the purpose of chemotherapy for solid cancer is to relieve symptoms and prolong life, it is determined to be effective as a drug effect even in a case where the curing effect is SD.

### (Pancreatic cancer patient 1)

In a pancreatic cancer patient 1 to which the A preparation was administered at 1.2 mg/m² as the dose of gemcitabine per administration, a tumor reducing effect of 30% or more was observed, and SD for 20 weeks was maintained.

This patient had received, as pre-curing, chemotherapy with gemcitabine and a combination therapy with gemcitabine and cisplatin. The patient was 67 years old and was female.

### (Pancreatic cancer patient 2)

In a pancreatic cancer patient 2 to which the A preparation was administered at 4.8 mg/m² as the dose of gemcitabine per administration, as a result of determining the tumor reducing effect on each of the days after 4 weeks and 8 weeks from the administration, it was determined to be PR.

This patient had received, as pre-curing, chemotherapy by a combination therapy with gemcitabine and capecitabine. The patient was 57 years old and was male.

### (Uterine cancer patient 1)

In a uterine cancer patient 1 to which the A preparation was administered at 1.2 mg/m² as the dose of gemcitabine per administration, it was determined to be SD in which the size of the tumor was not changed for 24 weeks. The patient was 73 years old and was female.

### (Pancreatic cancer patient 3)

In a pancreatic cancer patient 3 to which the A preparation was administered at 12 mg/m² as the dose of gemcitabine per administration, it was determined to be SD in which the size of the tumor was not changed for 8 weeks.

### (Pancreatic cancer patient 4)

In a pancreatic cancer patient 4 to which the A preparation was administered at 17 mg/m² as the dose of gemcitabine per administration, it was determined to be SD in which the size of the tumor was not changed for 8 weeks.

### (Liposarcoma patient 1)

In a liposarcoma patient 1 to which the A preparation was administered at 30 mg/m² as the dose of gemcitabine per administration, it was determined to be SD in which the size of the tumor was not changed for 12 weeks or more.

### (Uterine cancer patient 2)

In a uterine cancer patient 2 to which the A preparation was administered at 30 mg/m² as the dose of gemcitabine per administration, it was determined to be SD in which the size of the tumor was not changed for 8 weeks or more.

### <Example 2 (Reference): Administration test 2>

21 days were set as one cycle, the A preparation was administered to a cancer patient on the 1st day and the 8th day, and the cycle consisting of these 21 days was repeated.

The curing effect was determined according to the same criteria as in Example 1.

### (Pancreatic cancer patient 5)

In a pancreatic cancer patient 5 to which the A preparation was administered at 12 mg/m² as the dose of gemcitabine per administration, it was determined to be SD in which the size of the tumor was not changed for 10 weeks or more.

### (Appendix cancer patient 1)

In an appendix cancer patient 1 to which the A preparation was administered at 12 mg/m² as the dose of gemcitabine per administration, it was determined to be SD in which the size of the tumor was not changed for 10 weeks or more.

### (Lung cancer patient 1)

The A preparation was administered at 23 mg/m² as a dose of gemcitabine per administration in the first cycle, and the administration was discontinued for 13 days from the 1st day (the 22nd day from the first administration) of the second cycle. Then, on the 15th day (the 36th day from the first administration) of the second cycle, in a lung cancer (non-small cell lung cancer) patient 1 to which the A preparation was administered at 17 mg/m² as a dose of gemcitabine per administration, it was determined to be SD in which the size of the tumor was not changed for 8 weeks or more from the first administration.

### <Example 3: Administration test 3>

28 days were set as one cycle, the A preparation was administered to a cancer patient on the 1st day, and the cycle consisting of these 28 days was repeated.

The curing effect was determined according to the same criteria as in Example 1.

### (Biliary tract cancer patient 1)

In a biliary tract cancer patient 1 to which the A preparation was administered at 40 mg/m² as the dose of gemcitabine per administration, as a result of determining the tumor reducing effect after 8 weeks from the administration, it was determined to be PR. Then, it was also PR in the determination after 16 weeks from the administration.

### (Bladder cancer patient 1)

In a bladder cancer patient 1 to which the A preparation was administered at 40 mg/m² as the dose of gemcitabine per administration, it was determined to be SD in which the size of the tumor was not changed for 16 weeks or more.

### <Example 4: Administration test 4>

21 days were set as one cycle, the A preparation was administered to a cancer patient on the 1st day, and the cycle consisting of these 21 days was repeated.

The curing effect was determined according to the same criteria as in Example 1.

### (Pancreatic cancer patient 6)

In a pancreatic cancer patient 6 to which the A preparation was administered at 40 mg/m² as the dose of gemcitabine per administration, it was determined to be SD in which the size of the tumor was not changed for 6 weeks or more.

An excellent anti-tumor effect was obtained from the anti-tumor agent according to the embodiment of the present invention. Specifically, it is an effect of reducing the volume of the cancer and an effect of not changing the size of the tumor. From these results, the anti-tumor agent according to the embodiment of the present invention is expected to prolong the progression-free survival time and the overall survival time of patients, and it has a very useful effect from the viewpoint of improving the QOL of patients.

The anti-tumor agent according to the embodiment of the present invention is useful since it exhibits an excellent anti-tumor effect.

## Claims

1. An anti-tumor agent for use in curing cancer in a human, the anti-tumor agent comprising:
a liposome containing an inner water phase; and
an aqueous solution dispersing the liposome, which constitutes an outer water phase,
wherein the liposome encompasses gemcitabine or a salt thereof,
a lipid constituting the liposome contains at least hydrogenated soybean phosphatidylcholine, 1,2-distearoyl-3-phosphatidylethanolamine-polyethylene glycol, and cholesterol,
the gemcitabine or the salt thereof encompassed in the liposome is administered at a dose rate of 40 mg/m² body surface area to 60 mg/m² body surface area in terms of gemcitabine per administration, and
the anti-tumor agent is repeatedly administered a plurality of times of single administration at every three weeks to four weeks.

2. The anti-tumor agent for use according to claim 1,
wherein a formulation form is a liquid medicinal preparation, and
a concentration of the gemcitabine or the salt thereof is 0.01 mg/mL to 10 mg/mL in terms of gemcitabine.

3. The anti-tumor agent for use according to claim 1 or 2,
wherein the cancer is an advanced solid cancer.

4. The anti-tumor agent for use according to claim 3,
wherein the solid cancer is at least one selected from pancreatic cancer, uterine cancer, appendix cancer, ovarian cancer, lung cancer, breast cancer, biliary tract cancer, bladder cancer, colon cancer, gastric cancer, or non-Hodgkin lymphoma.

5. The anti-tumor agent for use according to any one of claims 1 to 4,
wherein an average particle diameter of the liposomes is 5 nm to 100 nm.

6. The anti-tumor agent for use according to any one of claims 1 to 5,
wherein in terms of a blending rate to a whole of the lipid constituting the liposome, the hydrogenated soybean phosphatidylcholine has a blending rate of 50% by mass to 90% by mass, the 1,2-distearoyl-3-phosphatidylethanolamine-polyethylene glycol has a blending rate of 1% by mass to 50% by mass, and the cholesterol has a blending rate of 1% by mass to 20% by mass.

7. The anti-tumor agent for use according to any one of claims 1 to 6,
wherein an administration route is an intravenous administration.

8. The anti-tumor agent for use according to any one of claims 1 to 7,
wherein the anti-tumor agent is administered by infusion for 15 minutes to 240 minutes in a single administration.

9. An anti-tumor agent for use in curing cancer in a human, the anti-tumor agent comprising:
a liposome containing an inner water phase; and
an aqueous solution dispersing the liposome, which constitutes an outer water phase,
wherein the liposome encompasses gemcitabine or a salt thereof,
a lipid constituting the liposome contains at least hydrogenated soybean phosphatidylcholine, 1,2-distearoyl-3-phosphatidylethanolamine-polyethylene glycol, and cholesterol, and
a cycle as one cycle of 28 days, in which on 1st day, an amount of the gemcitabine or the salt thereof encompassed in the liposome is administered at a dose rate of 40 mg/m² body surface area to 60 mg/m² body surface area in terms of gemcitabine per administration and the administration is discontinued from 2nd day to 27th day, is repeated.

10. An anti-tumor agent for use in curing cancer in a human, the anti-tumor agent comprising:
a liposome containing an inner water phase; and
an aqueous solution dispersing the liposome, which constitutes an outer water phase,
wherein the liposome encompasses gemcitabine or a salt thereof,
a lipid constituting the liposome contains at least hydrogenated soybean phosphatidylcholine, 1,2-distearoyl-3-phosphatidylethanolamine-polyethylene glycol, and cholesterol, and
a cycle as one cycle of 21 days, in which on 1st day, an amount of the gemcitabine or the salt thereof encompassed in the liposome is administered at a dose rate of 40 mg/m² body surface area to 60 mg/m² body surface area in terms of gemcitabine per administration and the administration is discontinued from 2nd day to 20th day, is repeated.

## Patentansprüche

1. Anti-Tumormittel zur Verwendung bei der Heilung von Krebs in einem Menschen, wobei das Anti-Tumormittel umfasst:
ein Liposom, das eine innere Wasserphase enthält; und
eine wässrige Lösung, die das Liposom dispergiert, die eine äußere Wasserphase aufbaut,
worin das Liposom Gemcitabin oder ein Salz hiervon umfasst,
ein Lipid, das das Liposom aufbaut, zumindest hydriertes Soja-Phosphatidylcholin, 1,2-Distearoyl-3-phosphatidylethanolamin-Polyethylenglykol und Cholesterin umfasst,
das Gemcitabin oder das Salz hiervon, das in dem Liposom umfasst ist, in einer Dosierung von 40 mg/m² Körperoberfläche bis 60 mg/m² Körperoberfläche verabreicht wird, ausgedrückt als Gemcitabin je Verabreichung, und
das Anti-Tumormittel mehrfach wiederholt in Einzelverabreichungen alle drei Wochen bis vier Wochen verabreicht wird.

2. Anti-Tumormittel zur Verwendung gemäß Anspruch 1, worin die Formulierungsform eine flüssige medizinische Zubereitung ist und
die Konzentration des Gemcitabins oder des Salzes hiervon 0,01 mg/ml bis 10 mg/ml beträgt, ausgedrückt als Gemcitabin.

3. Anti-Tumormittel zur Verwendung gemäß Anspruch 1 oder 2,
worin der Krebs ein fortgeschrittener fester Krebs ist.

4. Anti-Tumormittel zur Verwendung gemäß Anspruch 3,
worin der feste Krebs zumindest einer ist, der ausgewählt ist aus Bauchspeicheldrüsenkrebs, Gebärmutterkrebs, Blinddarmkrebs, Eierstockkrebs, Lungenkrebs, Brustkrebs, Gallengangkrebs, Blasenkrebs, Dickdarmkrebs, Magenkrebs oder Non-Hodgkin-Lymphom.

5. Anti-Tumormittel zur Verwendung gemäß irgendeinem der Ansprüche 1 bis 4,
worin der mittlere Partikeldurchmesser der Liposome 5 nm bis 100 nm beträgt.

6. Anti-Tumormittel zur Verwendung gemäß irgendeinem der Ansprüche 1 bis 5,
worin, ausgedrückt als Mischungsanteil an dem gesamten Lipid, das das Liposom aufbaut, das hydrierte Soja-Phosphatidylcholin einen Mischungsanteil von 50 Masse-% bis 90 Masse-% aufweist, das 1,2-Distearoyl-3-phosphatidylethanolamin-Polyethylenglykol einen Mischungsanteil von 1 Masse-% bis 50 Masse-% aufweist, und das Cholesterin einen Mischungsanteil von 1 Masse-% bis 20 Masse-% aufweist.

7. Anti-Tumormittel zur Verwendung gemäß irgendeinem der Ansprüche 1 bis 6,
worin die Verabreichungsroute eine intravenöse Verabreichung ist.

8. Anti-Tumormittel zur Verwendung gemäß irgendeinem der Ansprüche 1 bis 7,
worin das Anti-Tumormittel in einer Einzelverabreichung durch Infusion für 15 Minuten bis 240 Minuten verabreicht wird.

9. Anti-Tumormittel zur Verwendung bei der Heilung von Krebs in einem Menschen, wobei das Anti-Tumormittel umfasst:
ein Liposom, das eine innere Wasserphase enthält; und
eine wässrige Lösung, die das Liposom dispergiert, die eine äußere Wasserphase aufbaut,
worin das Liposom Gemcitabin oder ein Salz hiervon umfasst,
ein Lipid, das das Liposom aufbaut, zumindest hydriertes Soja-Phosphatidylcholin, 1,2-Distearoyl-3-phosphatidylethanolamin-Polyethylenglykol und Cholesterin umfasst, und
ein Zyklus, als ein Zyklus von 28 Tagen, worin an dem 1. Tag die Menge an Gemcitabin oder des Salzes hiervon, die in dem Liposom umfasst ist, in einer Dosierung von 40 mg/m² Körperoberfläche bis 60 mg/m² Körperoberfläche, ausgedrückt als Gemcitabin, je Verabreichung, verabreicht wird, und die Verabreichung vom 2. bis zum 27. Tag ausgesetzt wird, wiederholt wird.

10. Anti-Tumormittel bei der Verwendung bei der Heilung von Krebs, in einem Menschen, wobei das Anti-Tumormittel umfasst:
ein Liposom, das eine innere Wasserphase enthält; und
eine wässrige Lösung, die das Liposom dispergiert, die eine äußere Wasserphase aufbaut,
worin das Liposom Gemcitabin oder ein Salz hiervon umfasst,
ein Lipid, das das Liposom aufbaut, zumindest hydriertes Soja-Phosphatidylcholin, 1,2-Distearoyl-3-phosphatidylethanolamin-Polyethylenglykol und Cholesterin umfasst, und
ein Zyklus, als ein Zyklus von 21 Tagen, worin an dem 1. Tag die Menge von Gemcitabin oder des Salzes hiervon, die in dem Liposom umfasst ist, in einer Dosierung von 40 mg/m² Körperoberfläche bis 60 mg/m² Körperoberfläche, ausgedrückt als Gemcitabin, je Verabreichung, verabreicht wird, und die Verabreichung vom 2. bis zum 20. ausgesetzt wird, wiederholt wird.

## Revendications

1. Agent antitumoral destiné à être utilisé pour traiter un cancer chez un être humain, l'agent antitumoral comprenant :
un liposome contenant une phase aqueuse interne ; et
une solution aqueuse dispersant le liposome, qui constitue une phase aqueuse externe,
dans lequel le liposome renferme de la gemcitabine ou un sel de celle-ci,
un lipide constituant le liposome contient au moins de la phosphatidylcholine de soja hydrogénée, du 1,2-distéaroyl-3-phosphatidyléthanolamine-polyéthylèneglycol, et du cholestérol,
la gemcitabine ou le sel de celle-ci enfermé dans le liposome est administré(e) à un taux de dose de 40 mg/m² de surface corporelle à 60 mg/m² de surface corporelle en termes de gemcitabine par administration, et
l'agent antitumoral est administré de manière répétée une pluralité de fois en une seule administration toutes les trois à quatre semaines.

2. Agent antitumoral pour une utilisation selon la revendication 1, dans lequel une forme de formulation est une préparation médicinale liquide, et une concentration de la gemcitabine ou du sel de celle-ci est de 0,01 mg/ml à 10 mg/ml en termes de gemcitabine.

3. Agent antitumoral pour une utilisation selon la revendication 1 ou la revendication 2, dans lequel le cancer est un cancer solide avancé.

4. Agent antitumoral pour une utilisation selon la revendication 3, dans lequel le cancer solide est au moins un sélectionné parmi un cancer du pancréas, un cancer de l'utérus, un cancer de l'appendice, un cancer de l'ovaire, un cancer du poumon, un cancer du sein, un cancer des voies biliaires, un cancer de la vessie, un cancer du côlon, un cancer gastrique, ou un lymphome non hodgkinien.

5. Agent antitumoral pour une utilisation selon l'une quelconque des revendications 1 à 4, dans lequel le diamètre particulaire moyen des liposomes est de 5 nm à 100 nm.

6. Agent antitumoral pour une utilisation selon l'une quelconque des revendications 1 à 5, dans lequel en termes de taux de mélange par rapport à l'ensemble du lipide constituant le liposome, la phosphatidylcholine de soja hydrogénée présente un taux de mélange de 50 % en masse à 90 % en masse, le 1,2-distéaroyl-3-phosphatidyléthanolamine-polyéthylèneglycol présente un taux de mélange de 1 % en masse à 50 % en masse, et le cholestérol présente un taux de mélange de 1 % en masse à 20 % en masse.

7. Agent antitumoral pour une utilisation selon l'une quelconque des revendications 1 à 6, dans lequel une voie d'administration est une administration intraveineuse.

8. Agent antitumoral pour une utilisation selon l'une quelconque des revendications 1 à 7, dans lequel l'agent antitumoral est administré par perfusion pendant 15 minutes à 240 minutes en une seule administration.

9. Agent antitumoral destiné à être utilisé pour traiter un cancer chez un être humain, l'agent antitumoral comprenant :
un liposome contenant une phase aqueuse interne ; et
une solution aqueuse dispersant le liposome, qui constitue une phase aqueuse externe,
dans lequel le liposome renferme de la gemcitabine ou un sel de celle-ci,
un lipide constituant le liposome contient au moins de la phosphatidylcholine de soja hydrogénée, du 1,2-distéaroyl-3-phosphatidyléthanolamine-polyéthylèneglycol, et du cholestérol, et
un cycle, d'une durée de 28 jours, au cours duquel, le 1er jour, une quantité de la gemcitabine ou du sel de celle-ci enfermé dans le liposome est administrée à un taux de dose de 40 mg/m² de surface corporelle à 60 mg/m² de surface corporelle en termes de gemcitabine par administration et l'administration est interrompue du 2ème jour au 27ème jour, est répété.

10. Agent antitumoral destiné à être utilisé pour traiter un cancer chez un être humain, l'agent antitumoral comprenant :
un liposome contenant une phase aqueuse interne ; et
une solution aqueuse dispersant le liposome, qui constitue une phase aqueuse externe,
dans lequel le liposome renferme de la gemcitabine ou un sel de celle-ci,
un lipide constituant le liposome contient au moins de la phosphatidylcholine de soja hydrogénée, du 1,2-distéaroyl-3-phosphatidyléthanolamine-polyéthylèneglycol, et du cholestérol, et
un cycle, d'une durée de 21 jours, au cours duquel, le 1er jour, une quantité de la gemcitabine ou du sel de celle-ci enfermé dans le liposome est administrée à un taux de dose de 40 mg/m² de surface corporelle à 60 mg/m² de surface corporelle en termes de gemcitabine par administration et l'administration est interrompue du 2ème jour au 20ème jour, est répété.
